# EUROPÄISCHE PATENTANMELDUNG

(11) **EP 1 512 382 A1**
(43) Veröffentlichungstag der Anmeldung: **09.03.2005**
(21) Anmeldenummer: 04020187.3
(22) Anmeldetag: 25.08.2004
(51) Int. Cl.: A61F 2/16

(54) **Vorrichtung zum Implantieren einer Intraokularlinse**

(30) Priorität: 27.08.2003 DE 20313283 U
(71) Anmelder: *Acri.Tec Gesellschaft für ophthalmologische Produkte mbH, 16761 Hennigsdorf (DE)
(72) Erfinder: Lingenfelder, Chrisian, 89081 Ulm (DE)
(74) Vertreter: Eisenführ, Speiser & Partner

(57) **Zusammenfassung**

Eine Vorrichtung zum Implantieren einer Intraokularlinse in ein Auge mit einer Injektorhülse 1, einem Stößel 9, welcher in einer Führungsbohrung der Injektorhülse 1 axial verschiebbar angeordnet ist, und einem Linsenaufnahmeraum 4 in der Injektorhülse, in welchem, ausgerichtet mit der axialen Bewegungsrichtung des Stößels, die zu implantierende Intraokularlinse anzuordnen ist, wobei in der Hülsenwand der Injektorhülse 1 zumindest im Bereich des Linsenaufnahmeraumes 4 wenigstens eine Öffnung 5, 6 vorgesehen ist, welche von der Außenseite der Hülsenwand bis in den Linsenaufnahmeraum 4 sich erstreckt.

## Beschreibung

Die Erfindung betrifft eine Vorrichtung zum Implantieren einer Intraokularlinse in einem Auge mit einer Injektorhülse, einem Stößel, welcher in einer Führungsbohrung der Injektorhülse axial verschiebbar angeordnet ist, und einem Linsenaufnahmeraum in der Injektorhülse, in welchem, ausgerichtet mit der axialen Bewegungsrichtung des Stößels, die zu implantierende Intraokularlinse anzuordnen ist.

Eine derartige Vorrichtung ist aus US 4,681,102 bekannt. Bei der bekannten Vorrichtung wird mit Hilfe einer Faltkartusche die zu implantierende Linse in den Linsenaufnahmeraum der Injektorhülse eingesetzt, wobei flügelförmige Ansätze der Kartusche durch eine schlitzförmige Öffnung in der Hülsenwand der Injektorhülse ragen und dort gegen Verdrehen stabilisiert sind. Die zu implantierende Linse kann als faltbare Linse in der Kartusche aufgenommen werden. Im Linsenaufnahmenraum ist die zu implantierende Linse mit der axialen Bewegungsrichtung des Stößels ausgerichtet und wird beim Implantieren durch den Stößel durch das distale Ende der Kartusche, welche in eine Injektionskanüle mündet, geschoben. Die Injektionskanüle wird bei der Implantation durch einen Schnitt in das Auge eingesetzt und beim Verschieben des Stößels wird die Intraokularlinse durch die Injektionskanüle in das Auge implantiert. Schwierigkeiten bereit die Reinigung, insbesondere die maschinelle Reinigung derartiger Implantationsvorrichtungen.

Aufgabe der Erfindung ist es daher eine Vorrichtung der eingangsgenannten Art zu schaffen, welche insbesondere maschinell rückstandsfrei gereinigt werden kann.

Diese Aufgabe wird erfindungsgemäß dadurch gelöst, dass in der Hülsenwand der Injektorhülse zumindest im Bereich des Linsenaufnahmeraumes wenigstens eine Öffnung vorgesehen ist. Ferner können außerhalb des Bereiches des Linsenaufnahmeraums in der Hülsenwand Öffnungen vorgesehen sein, welche von der Außenseite der Hülsenwand bis zur Führungsbohrung sich erstrecken.

Die Öffnungen können schlitzförmig und/oder als zylindrische Bohrungen ausgebildet sein.

Vorzugsweise wird eine achsparallel sich erstreckende schlitzförmige Öffnung vorgesehen, die im Bereich des Linsenaufnahmeraumes angeordnet ist.

Bei einem bevorzugten Ausführungsbeispiel ist der Linsenaufnahmeraum durch einen in der Hülsenwand axial sich erstreckenden Einschubschlitz zum Einsetzen der zu implantierenden Linse, insbesondere mit Hilfe einer Kartusche, ausgestattet, wobei bei diesem Ausführungsbeispiel die achsparallele schlitzförmige Öffnung diametral zum Einschubschlitz in der Hülsenwand angeordnet ist.

Durch die vorzugsweise als zylindrische Bohrungen ausgebildeten Öffnungen in der Injektorhülse und die schlitzförmige Öffnung wird ein wirkungsvolles Durchspülen der Vorrichtung ermöglicht. Man erreicht dadurch eine rückstandsfreie maschinelle Reinigung.

Anhand der Figuren wird an einem Ausführungsbeispiel die Erfindung noch näher erläutert.

Es zeigt:
- Figur 1: einen Längsschnitt durch das Ausführungsbeispiel; und
- Figur 2: eine Draufsicht auf das Ausführungsbeispiel.

Das in den Figuren dargestellte Ausführungsbeispiel beinhaltet eine Injektorhülse 1. Diese besteht aus einer Innenhülse 17 und einer Außenhülse 16, die ineinander geschoben sind. Am proximalen Ende der Außenhülse 16 ist mit einer Befestigungsschraube 13 ein Griffstück 11 befestigt.

In einer axialen Führungsbohrung 3 der Injektorhülse 1 ist ein Führungskolben 2 axial verschiebbar geführt. Am proximalen Ende des Führungskolbens 2 ist ein Betätigungsknopf 10 befestigt. Mit Hilfe des Betätigungsknopfes 10 wird der Führungskolben 2 in der Führungsbohrung 3 in axialer Richtung hin und her verschoben.

Am distalen Ende des Führungskolbens 2 ist ein Stößel 9 befestigt. Durch das Verschieben des Führungskolben 2 wird der Stößel 9 ebenfalls in axialer Richtung, d. h. entlang einer Hülsenachse 19 verschoben.

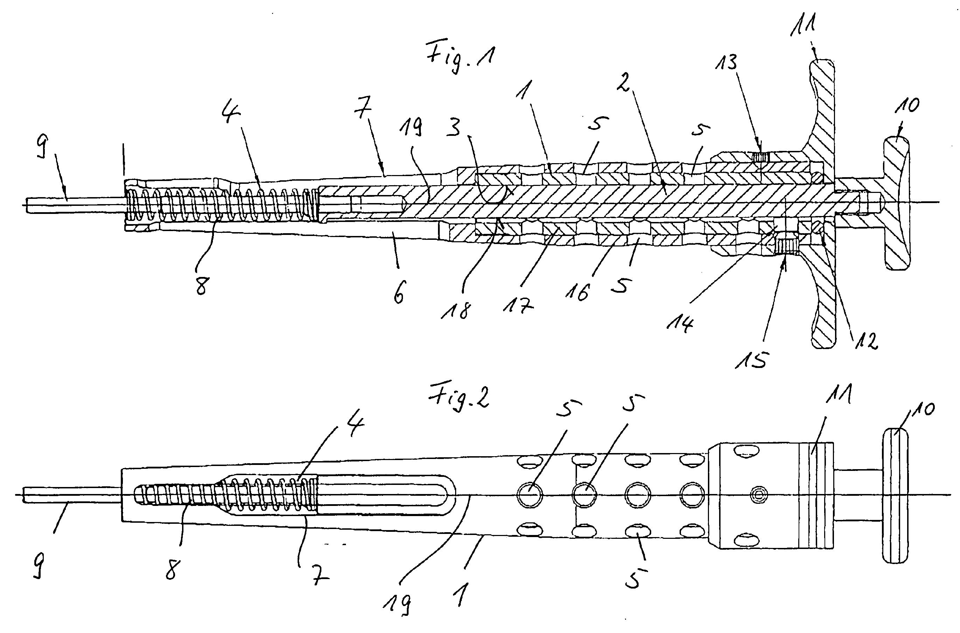

Im Innern der Injektorhülse 1 befindet sich ein Linsenaufnahmeraum 4. Eine zu implantierende Intraokularlinse kann durch einen Einschubschlitz 7 in der Hülsenwand der Injektorhülse 1 in den Linsenaufnahmeraum 4 eingebracht werden, nachdem der Führungskolben 2 und damit der Stößel 9 in eine zurückgezogene Stellung (in den Figuren 1 und 2 nach rechts) gebracht worden sind. Hierbei wird gleichzeitig eine Druckfeder 8 zur Freigabe des Linsenaufnahmeraumes 4 zurückgezogen. Die zu implantierende Intraokularlinse kann durch den Einschubschlitz 7 mit Hilfe einer Pinzette oder mit Hilfe einer Faltkartusche, wie sie beispielsweise aus US 4,681,102 bekannt ist, in den Linsenaufnahmeraum 4 eingesetzt werden. Die im Linsenaufnahmeraum 4 eingesetzte Intraokularlinse, welche sich vorzugsweise in gefaltetem oder gerolltem Zustand befindet, ist mit der Bewegungsrichtung des Stößels 9 ausgerichtet und wird bei Betätigung durch den Betätigungskopf 10 aus dem Linsenaufnahmeraum 4 in den Figuren 1 und 2 nach links geschoben und gelangt über eine nicht näher dargestellte Injektionskanüle, die in das Auge eingesetzt ist, in das Augeninnere.

Diese Vorschubbewegung erfolgt gegen die Kraft der Druckfeder 8. Die Druckfeder kann auch an einer anderen geeigneten Stelle vorgesehen sein, an der sie zwischen dem Stößel 9 und der Injektorhülse 1 wirksam ist.

Am Führungskolben 2 ist eine ebene Kolbenfläche 18 vorgesehen. Diese ebene Kolbenfläche 18 kann entlang einer gegebenenfalls keilförmig ausgebildeten Verdrehsicherung 14 mit vorzugsweise ebenfalls ebener Anlagefläche entlanggeführt werden. Die Verdrehsicherung 14 ist ferner durch eine Blockierschraube 15 an der Injektorhülse fixiert. Hierdurch ist auch eine axiale Fixierung des Führungskolbens 2 möglich. Die Abdichtung nach außen erfolgt mittels eines am proximalen Ende zwischen der Injektorhülse 2 und dem Griffstück 11 angeordneten O-Ringes 12.

In der Hülsenwand der Injektorhülse 1 sind kreiszylindrische Bohrungen als Öffnungen 5, welche sich durch die Hülsenwand erstrecken, vorgesehen. Wie aus der Figur 1 zu ersehen ist, erstrecken sich die Bohrungen 5 sowohl durch die Außenhülse 16 als auch durch die Innenhülse 17 bis in die Führungsbohrung 3 im Innern der Injektorhülse 1.

Ferner ist diametral gegenüber dem Einschubschlitz 7 eine in axialer Richtung sich erstreckende schlitzförmige Öffnung 6 in der Hülsenwand der Injektorhülse 1 vorgesehen. Hierdurch wird vor allem im Bereich des Linsenaufnahmeraumes 4 eine wirkungsvolle Durchspülung beim dargestellten Ausführungsbeispiel erreicht. Dabei erreicht man insbesondere eine rückstandsfreie Reinigung an der Druckfeder und am Stößel 9 und insbesondere in den Bereichen, die sich im Linsenaufnahmeraum 4 befinden. Durch die als Bohrungen ausgebildeten Öffnungen 5 wird eine Reinigung im Bereich des Führungskolbens 2 erreicht.

### [Bezugszeichenliste]

- 1: Injektorhülse
- 2: Führungskolben
- 3: Führungsbohrung
- 4: Linsenaufnahmeraum
- 5: Öffnungen (Bohrungen)
- 6: schlitzförmige Öffnung
- 7: Einschubschlitz
- 8: Druckfeder
- 9: Stößel
- 10: Betätigungsknopf
- 11: Griffstück
- 12: O-Ring
- 13: Befestigungsschraube
- 14: Verdrehsicherung
- 15: Blockierschraube
- 16: Außenhülse
- 17: Innenhülse
- 18: ebene Kolbenfläche
- 19: Hülsenachse

## Patentansprüche

1. Vorrichtung zum Implantieren einer Intraokularlinse in ein Auge mit einer Injektorhülse, einem Stößel, welcher in einer Führungsbohrung der Injektorhülse axial verschiebbar angeordnet ist, und einem Linsenaufnahmeraum in der Injektorhülse, in welchem, ausgerichtet mit der axialen Bewegungsrichtung des Stößels, die zu implantierende Intraokularlinse anzuordnen ist,
**dadurch gekennzeichnet, dass** in der Hülsenwand der Injektorhülse (1) zumindest im Bereich des Linsenaufnahmeraumes (4) wenigstens eine Öffnung (5, 6) vorgesehen ist, welche von der Außenseite der Hülsenwand bis in den Linsenaufnahmeraum (4) sich erstreckt.

2. Vorrichtung nach Anspruch 1,
**dadurch gekennzeichnet, dass** die wenigstens eine Öffnung (5, 6) schlitzförmig ausgebildet ist.

3. Vorrichtung nach Anspruch 1 oder 2,
**dadurch gekennzeichnet, dass** der Linsenaufnahmeraum (4) in der Hülsenwand einen sich axial erstreckenden Einschubschlitz (7) zum Einsetzen der zu implantierenden Linse in den Linsenaufnahmeraum (4) aufweist und das die achsparallele, schlitzförmige Öffnung (6) diametral zum Einschubschlitz (7) in der Hülsenwand angeordnet ist.

4. Vorrichtung nach einem der Ansprüche 1 bis 3,
**dadurch gekennzeichnet, dass** in der Hülsenwand der Injektorhülse (1) ferner Öffnungen (5) in Form von Bohrungen vorgesehen sind, welche sich von der Außenseite bis zur Führungsbohrung (3) in dem außerhalb des Linsenaufnahmeraums (4) befindlichen Bereich der Hülsenwand erstrecken.
